**Europäisches Patentamt**

**European Patent Office**

**Office européen des brevets**

(11) Veröffentlichungsnummer : **0 407 904 B1**

## (12) EUROPÄISCHE PATENTSCHRIFT

(45) Veröffentlichungstag der Patentschrift :
**21.09.94 Patentblatt 94/38**

(51) Int. Cl.$^5$ : **G01N 33/543, // G01N33/563**

(21) Anmeldenummer : **90112911.4**

(22) Anmeldetag : **06.07.90**

(54) **Verfahren zur Bestimmung eines Analyten.**

(30) Priorität : **12.07.89 DE 3922960**

(43) Veröffentlichungstag der Anmeldung :
**16.01.91 Patentblatt 91/03**

(45) Bekanntmachung des Hinweises auf die
Patenterteilung :
**21.09.94 Patentblatt 94/38**

(84) Benannte Vertragsstaaten :
**AT BE CH DE DK ES FR GB GR IT LI LU NL SE**

(56) Entgegenhaltungen :
**EP-A- 0 173 375**

(56) Entgegenhaltungen :
**WO-A-82/01072**
**US-A- 4 469 787**
**US-A- 4 829 011**
**E. ISHIKAWA et al., (Eds.), "Enzyme Immu-noassays", 1981, Igaku-Shoin, Tokyo (JP)**

(73) Patentinhaber : **BOEHRINGER MANNHEIM GMBH**
**D-68298 Mannheim (DE)**

(72) Erfinder : **Mangold, Dieter, Dr.**
**Hüttenmüllerstrasse 33**
**D-6701 Maxdorf (DE)**
Erfinder : **Schlipfenbacher, Reiner, Dr.**
**Beethovenstrasse 9**
**D-6840 Lampertheim (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist ein Verfahren zur Bestimmung eines Analyten sowie ein Reagenz, welches in diesem Verfahren eingesetzt werden kann.

Die Bestimmung von Analyten ist insbesondere in der klinischen Diagnostik ein weitverbreitetes Anliegen. In jüngerer Zeit wurden dazu wegen der größen erreichbaren Genauigkeit sowie der breiten Anwendbarkeit insbesondere Verfahren angewandt, die immunologische Reaktionsschritte beinhalten. Wegen des Vorteils der leichten Handhabbarkeit geht man immer mehr dazu über, solche Verfahren unter Zuhilfenahme eines an einen festen Träger gebundenen Bestandteils einer immunologischen Reaktion durchzuführen. Beispielsweise ermöglichen diese Verfahren die Verwendung von Teststreifen, auf denen eine ganze Reaktionssequenz allein durch Inkontaktbringen der Probe mit dem Streifen abläuft.

Immunologische Bestimmungsverfahren können aufgrund der Art der beteiligten Reaktionspartner in verschiedene Klassen eingeteilt werden. Eine davon ist die Klasse der sogenannten kompetitiven Verdrängungstests. Dabei wird ein immobilisiertes Antigen, an welches ein markierter Antikörper gebunden ist, mit der Probe in Kontakt gebracht. Das immobilisierte Antigen wird bei Anwesenheit des Analyten in einer Gleichgewichtsreaktion aus dem Immunkomplex mit dem markierten Antikörper verdrängt. Der ehemals immobilisierte markierte Antikörper geht somit als Komplex mit dem zu bestimmenden Analyten in die flüssige Phase über und kann nach Abtrennung der flüssigen Phase von der festen Phase über seine Markierung bestimmt werden. Daraus läßt sich die Konzentration des Analyten in der Probe ermitteln.

Ein solcher Immuntest ist beispielsweise in der EP-A-0173375 beschrieben. Als markierter Antikörper wird ein markiertes Fab-Fragment eingesetzt, der zu Beginn des Tests im Komplex mit immobilisiertem Analyten vorliegt.

In der US-A-4436236 ist ein ähnliches Verfahren geschildert, es wird jedoch ein immobilisiertes Antigen verwendet, welches eine geringere Affinität zu dem markierten Antikörper aufweist als der Analyt. Auch in diesem Verfahren werden bevorzugt markierte Antikörperfragmente eingesetzt.

Die Verfahren der EP-A-0173375 und der US-A-4436236 haben den Nachteil, daß sie hohe Leerwerte der Messung aufweisen, selbst wenn kein Analyt in der Probe ist, die Signalgröße jedoch relativ gering ist.

In der US-A-4277560 wird ein Immunoassay beschrieben, bei dem ein markierter Analyt über einen immobilisierten Antikörper reversibel an eine Festphase gebunden ist. Der markierte Analyt wird durch den in der Probe enthaltenen Analyten von der Festphase verdrängt und kann danach als Maß für die Menge an zu bestimmenden Analyten verwendet werden. Dieses Verfahren hat den Nachteil, daß die Genauigkeit der Ergebnisse sehr stark von der Gleichmäßigkeit der Beladung der festen Phase abhängt. Eine genügende Genauigkeit ist nur schwer zu erreichen.

Aufgabe der vorliegenden Erfindung war es daher, ein Verfahren zur Bestimmung eines Analyten bereitzustellen, bei dem die Nachteile der Verfahren des Standes der Technik, insbesondere relativ hohe Leerwerte und aufwendige Maßnahmen bei der Herstellung, vermieden werden. Das Verfahren sollte genau und leicht automatisierbar sein.

Die Aufgabe wird gelöst durch ein Verfahren zur Bestimmung eines Analyten in einer flüssigen Probe durch Inkontaktbringen der Probenflüssigkeit mit einem immobiliserten Analyten oder Analytanalogen und einem markierten Antikörper, der sowohl mit dem zu bestimmenden Analyten, als auch mit dem immobilisierten Analyten oder immobilisierten Analytanalogen eine immunologische Reaktion eingehen kann und der durch Bindung an den immobilisierten Analyten bzw. das immobilisierte Analytanalogon immobilisiert ist, dadurch gekennzeichnet, daß der markierte Antikörper pro Marker mindestens 4 Bindungsstellen für den Analyten bzw. das Analytanaloge aufweist.

Ebenfalls Gegenstand der Erfindung ist ein Reagenz zur Durchführung des genannten Verfahrens.

Der Analyt, der in dem erfindungsgemäßen Verfahren bestimmt werden kann, ist insbesondere ein Antigen oder ein Hapten. Die Bestimmung kann qualitativ erfolgen, also um festzustellen, ob der Analyt in der Probe vorhanden ist. Sie kann jedoch auch quantitativ zur Bestimmung der Konzentration oder Menge eines Analyten in der Probe genutzt werden. Die flüssige Probe ist bevorzugt eine wässrige Lösung, Suspension oder Emulsion. Besonders bevorzugt sind Körperflüssigkeiten oder davon abgeleitete Flüssigkeiten, wie Blut, Serum, Plasma oder Harn.

Die Konzentration des Analyten kann mit dem vorgeschlagenen Verfahren in einem Bereich von $10^{-9}$ bis zu $10^{-5}$ mol/l , bevorzugt von $10^{-8}$ bis zu $10^{-6}$ mol/l bestimmt werden. Ganz besonders bevorzugt ist die Bestimmung sogenannter hochkonzentrierter Analyten; als solche bezeichnet man Analyten im Konzentrationsbereich größer als $10^{-8}$ mol/l. Beispiele solcher Analyten in Harn sind Albumin oder $a_1$ Mikroglobulin ($a_1$M).

Unter einem Analytanalogen ist eine dem Analyten immunologisch nahestehende Verbindung zu verstehen. Das Analoge unterscheidet sich zwar strukturell etwas vom Analyten, wird jedoch von einem Antikörper gegen den Analyten erkannt. Beispielsweise können im Sinne der vorliegenden Erfindung solche Analytanaloga verwendet werden, die von dem markierten Antikörper starker gebunden werden als der Analyt selbst. In diesem Fall ist der Leerwert, also das Meßergebnis ohne Vorhandensein des Analyten besonders niedrig. Beispiel für ein solches Analyt/Analytanalogonpaar ist: Schweine-Albumin/Human-Albumin, Affen-Albumin/Human-Albumin.

Immobilisierte Analyten bzw. Analytanaloga sind an eine feste Phase gebundene Analyten bzw. Analytanaloga. Die Bindung kann kovalent, präzipitativ, über spezifische Wechselwirkungen oder adsorptiv sein. Jede dieser Bindungsarten kann verwendet werden, wenn gewährleistet ist, daß sich der Analyt bzw. das Analytanalogon nicht in wesentlichem Ausmaß von der festen Phase ablöst. Zu jeder der Bindungsarten stehen dem Fachmann bekannte Methoden zur Verfügung. Die Art der festen Phase richtet sich nach der Art der Bindung des Analyten bzw. Analytanalogen. Ist der Analyt kovalent gebunden, so muß beispielsweise eine feste Phase verwendet werden, die reaktive Gruppen aufweist. Bei der Bindung über spezifische Wechselwirkungen ist die Bindung über Biotin/Streptavidin bevorzugt. Beispielsweise wird dann die feste Phase mit einem Streptavidin enthaltenden Überzug versehen und der daran gebundene Analyt, bzw. das Analog an Biotin kovalent gebunden. Es bildet sich eine recht feste Bindung über Streptavidin und Biotin. Die feste Phase kann in Form von Partikeln, Papieren, Vliesmaterialien, Membranen, Geweben aber auch Küvetten, Mikrotiterplatten etc. vorliegen. Soll das Nachweisverfahren auf einem Teststreifen durchgeführt werden, so sind saugfähige Vliese bevorzugt.

Markierter Antikörper ist im erfindungsgemäßen Verfahren ein Antikörper, der sowohl mit dem zu bestimmenden Analyten als auch dem immobilisierten Analyten bzw. Analytanalogen eine immunologische Reaktion eingehen kann. Antikörper gegen den Analyten bzw. das Analytanalogon können durch bekannte Verfahren hergestellt und selektiert werden. Verwendet werden können sowohl polyklonale als auch monoklonale Antikörper, wobei die monoklonalen bevorzugt sind.

Als Markierung kann jede Substanz dienen, mit Hilfe derer die Anwesenheit des Antikörpers quantitativ oder qualitativ nachgewiesen werden kann. Eine geeignete Markierung ist beispielsweise ein Enzym, ein Metall, ein Rest, dessen Emission oder Absorption von Licht oder radioaktiver Strahlung gemessen werden kann, oder ein Rest, der durch chemische oder immunologische Reaktion in einen solchen Rest überführt werden kann. Dem Fachmann steht eine größe Auswahl von Markierungen zur Verfügung. Bedingung für die Markierung ist, daß es möglich sein muß, an dieser Markierung mehrere Bindungsstellen eines Antikörpers zu befestigen. Die Markierung weist daher bevorzugt reaktive Gruppen, beispielsweise Hydroxy-, Amino-, Mercapto- oder Carboxylgruppen auf, welche mit Antikörpern direkt oder indirekt verknüpft werden können.

Enzyme, die als Markierung geeignet sind, sind bespielsweise Hydrolasen, wie $\beta$-Galaktosidase, oder Peroxidasen, wie POD.

Metalle, insbesondere solche in Form feinstverteilter Partikel, beispielsweise Kolloide, umfassen beispielsweise Gold. Solche Markierungen sind beispielsweise in der EP-A-0258963 beschrieben. Fluoreszierende Verbindungen sind beispielsweise Resorufine; farbige Verbindungen sind beispielsweise Phycoerythrin, gefärbte Latexpartikel und gefärbte Tellur und Selenoxide (EP-A 0298368). Bevorzugte Markierungsmittel sind Enzyme und Metalle, besonders bevorzugt Enzyme. Der markierte Antikörper des erfindungsgemäßen Verfahrens weist im Gegensatz zu den im Stand der Technik genannten Antikörpern mindestens 4, bevorzugt 6 bis 12 Bindungsstellen für den Analyten bzw. das Analytanalogon auf. Ein solcher markierter Antikörper wird im folgenden als oligovalenter markierter Antikörper bezeichnet.

Zur Herstellung des markierten oligovalenten Antikörpers aus Antikörpern und einer Markierung wird bevorzugt eine Markierung mit mehreren Antikörpern, die weniger Bindungsstellen haben, beispielsweise 1 oder 2 Bindungsstellen, wie Fab-Fragmente oder IgG, umgesetzt.

Bei den Verfahren zur Herstellung von markierten oligovalenten Antikörpern entstehen oft auch Gemische von markierten Antikörpern, die eine verschieden größe Anzahl von Bindungsstellen haben. Aus diesem Gemisch können Gemische von markierten Antikörpern isoliert werden, die ein Gemisch von markierten Antikörpern mit einer bestimmten Bindungsstellenzahl darstellen, beispielsweise mit 4 bis 7 Bindungsstellen pro Markierung. Diese Gemische können im erfindungsgemäßes Verfahren vorteilhaft eingesetzt werden, wenn sie überwiegend markierte Antikörper mit mindestens 4 Bindungsstellen pro Markierung enthalten.

Für enzymmarkierte Antikörper ist die Bindung von 5 IgG oder 10 Fab-Fragmenten an das Enzym besonders vorteilhaft.

Die Herstellung solcher markierter oligovalenter Antikörper ist bekannt und beispielsweise in Kitigawa in Enzyme Immunoassay (Eds. Ishikawa, Kuwai, Migui; Igaku Shoin Tokyo/New York (1981), S. 81-89) beschrieben.

Bevorzugt wird in dem erfindungsgemäßen Verfahren das Gemisch aus immobilisiertem Analyten bzw. Analytanalogon und markiertem Antikörper als immobilisierter Immunkomplex aus diesen Bestandteilen eingesetzt. Eine solche einen Immunkomplex enthaltende feste Phase, die den markierten Antikörper durch den Probenanalyten verdrängbar enthält, wird im folgenden auch Verdrängungsmatrix genannt.

EP 0 407 904 B1

Zur Herstellung wird aus dem bereits immobilisierten Analyten bzw. Analytanalogon und dem markierten Antikörper in einer immunologischen Reaktion ein immobilisierter Immunkomplex gebildet.

Eine weitere Methode oder Herstellung einer solchen Verdrängungsmatrix ist die Durchführung einer immunologischen Fällungsreaktion zwischen dem Analyten bzw. Analytanalogon und einem Antikörper gegen den Analyten bzw. das Analogon auf die feste Phase. Anschließend wird mit dem markierten Antikörper umgesetzt. Eine solche Methode ist beispielsweise in der EP-A-0 312 907 beschrieben.

Falls die Immobilisierung des Analyten bzw. des Analytanalogons über spezifische Wechselwirkungen, wie Biotin/Streptavidin erfolgen soll, kann ein biotinylierter Analyt oder biotinyliertes Analytanalogon in einer immunologischen Reaktion mit dem markierten Antikörper zu einem löslichen Immunkomplex umgesetzt und diese dann mit einer mit Streptavidin überzogenen festen Phase in Kontakt gebracht. Man erhält nach Abtrennung der flüssigen Phase auch ohne Waschschritte eine Verdrängungsmatrix, die praktisch nicht mit nicht komplexierten markierten Antikörpern bzw. überschüssiger Markierung verunreinigt ist. Es kann aber auch in einer ersten Reaktion der biotinylierte Analyt oder Analytanalogon mit der mit Streptavidin überzogenen festen Phase in Kontakt gebracht werden.

Als vorteilhaft haben sich insbesondere folgende Mengenverhältnisse der Bestandteile erwiesen: Je höher die zu bestimmende Analytkonzentration ist, desto höher sollte die Menge an immobilisiertem Analyten/Analytanalogon sein. Wird die Menge an markiertem Antikörper in Relation zu der Menge an Analyt bzw. Analytanalogon zu gering gewählt, dann wird das Verfahren in manchen Fällen schlechter brauchbar.

Die Menge an immobilisiertem Analyten bzw. Analytanalogon ist bevorzugt 1 ng - 0.1 mg/cm² Matrix, besonders bevorzugt 0.1 μg - 10μg/cm² Matrix. Die Menge an markiertem Antikörper beträgt 1 - 1000 mU/cm², besonders bevorzugt 10-500 mU/cm² Matrix.

Die Durchführung des erfindungsgemäßen Verfahrens erfolgt im allgemeinen analog zu den für kompetitive Verdrängungstests bekannten Prinzipien, jedoch unter Verwendung der erfindungsgemäßen Verdrängungsmatrix.

Das Verfahren ist besonders geeignet zur Bestimmung von kleinen Probenmengen. Geeignet zur Untersuchung mittels üblicher Teststreifen sind insbesondere Probenvolumina von 5 μl bis 1 ml. Die Volumina hängen von der Saugfähigkeit der verwendeten Matrices ab. Bevorzugt überschreiten die Probenvolumina die Saugvolumina der Matrices nicht.

Zu Beginn des erfindungsgemäßen Verfahrens wird das Probenvolumen mit dem immobilisierten Analyten oder Analytanalogen und dem markierten Antikörper in Kontakt gebracht und eine bestimmte Zeit dort belassen. Während dieser Zeit findet bei Anwesenheit des zu bestimmenden Analyten in der Probe die kompetitive Verdrängung des immobilisierten Analyten oder immobilisierten Analytanalogons aus dem Immunkomplex mit dem markierten Antikörper statt. Es bildet sich ein löslicher Immunkomplex aus Analyt und markiertem Antikörper. Je mehr Analyt in der Probe vorhanden ist, desto mehr des löslichen Immunkomplexes wird gebildet. Daher ist es möglich, die Menge des Analyten aus der Menge an auf der Festphase verbleibendem markierten Antikörpers oder des in der flüssigen Phase befindlichen markierten Antikörpers zu bestimmen. Dazu wird die flüssige Phase von der festen Phase zumindest teilweise getrennt. Dann wird die Menge an Markierung in bzw. an einer der beiden Phasen in üblicher Weise bestimmt. Ist die Markierung ein Enzym, wird die Phase unter Bedingungen, die für die Enzymreaktion geeignet sind, mit einem Substrat umgesetzt. Die Menge an umgesetztem Substrat ist ebenfalls ein Maß für die Menge an Analyt in der Probe.

Durch Ausführung des erfindungsgemäßen Verfahrens mit Proben bekannter Analytkonzentration erhält man eine Eichkurve, aus der die Konzentration des Analyten in einer Probe bisher nicht bekannten Analytgehalts aus den damit erhaltenen Meßwerten abgelesen werden kann.

Das Verfahren kann in verschiedenen Variationen durchgeführt werden:

In einer Ausführungsform wird in ein Eppendorfhütchen mit einem Testbezirk, der den immobilisierten Analyten bzw. das Analytanalogon und den enzymmarkierten Antikörper enthält, analythaltige Probe einpipettiert. Nach Schütteln, beispielsweise 5 Min. lang, wird ein Teil der Lösung in eine Küvette überführt, die ein chromogenes Substrat für das Enzym enthält.

Gemessen wird die Geschwindigkeit der Farbbildung durch Extinktionsmessung bei einer Wellenlänge, bei der das entstandene farbige Produkt Licht absorbiert.

In einer weiteren Ausführungsform wird eine Küvette, die einen oben beschriebenen Testbezirk aufweist, mit der Probe versetzt, die Mischung eine Zeitlang inkubiert und die flüssige Phase aus der Küvette entfernt. Es kann danach ein Waschschritt zur vollständigen Entfernung von Resten der flüssigen Phase angeschlossen werden. Anschließend wird eine Lösung eines chromogenen Substrats für das Markierungsenzym in die Küvette gegeben. Auch hier wird die Farbänderung gemessen. Im Gegensatz zu der oben geschilderten Ausführungsform ist jedoch die Farbänderung umso kleiner, je mehr Analyt in der Probe vorhanden war.

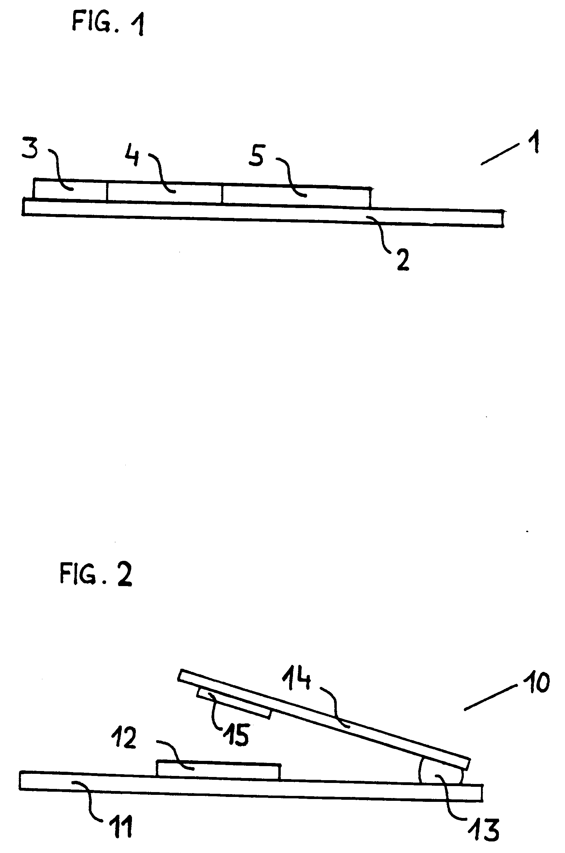

Eine besonders bevorzugte Ausführungsform ist ein Chromatographiestreifen gemäß Figur 1. Der Streifen 1 ist aufgebaut aus einer Grundfolie 2, auf der ein Saugvlies 3, eine Verdrängungsmatrix 4 und ein Substratvlies

4

saugfähig miteinander verbunden angebracht sind. Der Streifen 1 wird so in die Probenflüssigkeit gestellt, daß von den Vliesen nur Saugvlies 3 mit der Probe in Berührung kommt. Die Verwendung von Saugvliesen ist besonders vorteilhaft, jedoch nicht unbedingt erforderlich. Die Probe wird von dort aus in die Verdrängungsmatrix 4 gesaugt. Diese Verdrängungsmatrix ist beispielsweise ein saugfähiges Vlies, auf dem der Analyt oder das Analytanaloge immobilisiert sind und das den markierten Antikörper in Form eines Immunkomplexes mit dem immobilisierten Analyten oder Analytanalogen enthält. Der zu bestimmende Analyt verdrängt auch hier den immobilisierten Analyten bzw. das Analytanalogon aus dem Immunkomplex mit dem markierten Antikörper. Der dadurch entstandene lösliche Immunkomplex fließt mit der Probenflüssigkeit in die Zone 5, auf welcher das zu der Enzymmarkierung passende chromogene Substrat imprägniert vorliegt. In der Substratzone 5 wird die Farbänderung gemessen. Gewünschtenfalls kann zwischen Verdrängungsmatrix 4 und Substratzone ein langsam saugendes Gewebe zur Verzögerung des Flüssigkeitsstroms angebracht werden.

Eine weitere Ausführungsform ist ein Teststreifen 10 gemäß Figur 2.

Auf einer Grundfolie 11 ist eine Verdrängungsmatrix 12 befestigt, die den immobilisierten Analyten bzw. Analytanalogon sowie den markierten Antikörper enthält. An der Grundfolie ist beispielsweise über eine Klebestelle 13 eine mindestens teilweise transparente bewegliche Klappe 14 angebracht. Auf der der Verdrängungsmatrix zugewandten Seite befindet sich ein Film 15, der das chromogene Substrat enthält. Nach der Inkubationszeit wird der Film 15 durch Senken der Klappe auf die Matrix 12 gedrückt und dadurch die Bestimmungsreaktion gestartet. Ein Test mit diesem Teststreifen kann unter anderem sowohl photometrisch als auch bei zusätzlicher Verwendung von reflektierenden Bestandteilen reflektionsphotometrisch ausgewertet werden.

Das erfindungsgemäße Verfahren hat den Vorteil, daß der Leerwert im Vergleich zum Meßsignal relativ klein ist. Es hat sich außerdem herausgestellt, daß bei den aus dem Stand der Technik bekannten kompetitiven Verdrängungstests mit Verunreinigungen des eingesetzten markierten Antikörpers mit nicht bindenden Verunreinigungen, beispielsweise überschüssigem Markierungsmittel, gerechnet werden muß. Diese Verunreinigungen müssen beispielsweise durch Waschen der den Immunkomplex aus immobilisiertem Analyten bzw. Analytanalogon und markiertem Antikörper des Standes der Technik enthaltenden Verdränungsmatrix vor der Durchführung des Tests entfernt werden. Durch das günstige Verhältnis von Bindungsstellen zu Markierungsmittel im markierten Antikörper des erfindungsgemäßen Verfahrens wird eine Verunreinigung durch umgesetztes Markierungsmittel weitgehend vermieden. Ein weiterer Vorteil des erfindungsgemäßen Verfahrens ist, daß es besonders wenige Schritte und Matrices umfaßt. Dadurch entfallen aufwendige und kostensowie zeitintensive Stufen.

Ebenfalls Gegenstand der Erfindung ist ein Reagenz zur Durchführung des erfindungsgemäßen Verfahrens zur Bestimmung eines Analyten in einer Probe, das einen immobilisierten Analyten oder ein immobilisiertes Analytanalogon sowie einen markierten Antikörper, der sowohl mit dem zu bestimmenden Analyten als auch mit dem immobilisierten Analyten oder immobilisierten Analytanalogon eine immunologische Reaktion eingehen kann, wobei der markierte Antikörper pro Markierung mindestens vier Bindungsstellen für den Analyten bzw. das Analytanalogon aufweist.

Auf folgende Figuren wird Bezug genommen:

Fig. 1 zeigt den Längsschnitt durch einen Chromatographiestreifen.

Fig. 2 zeigt den Längsschnitt durch einen Teststreifen mit Klappe.

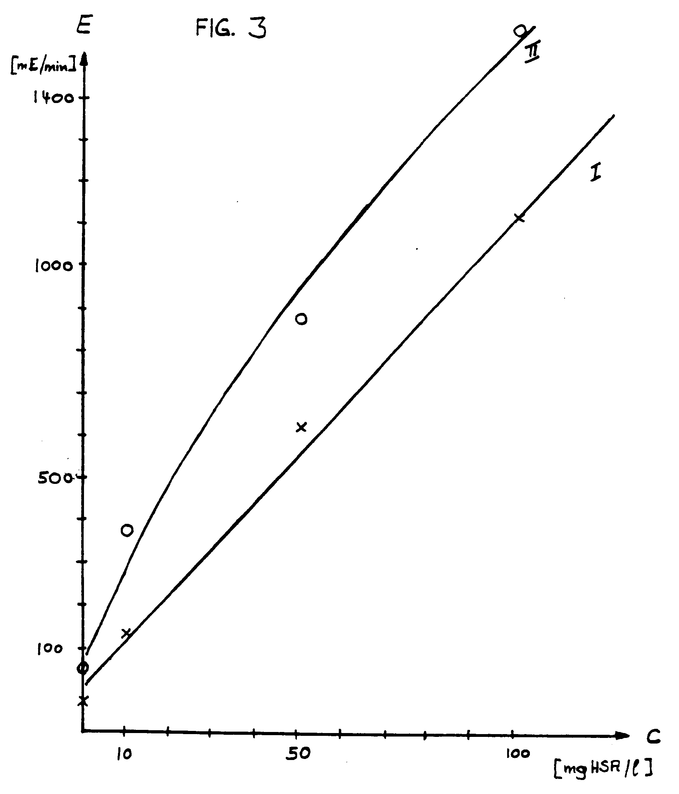

Fig. 3 zeigt eine Eichkurve zur Bestimmung von Albumin.

Die folgenden Beispiele erläutern die Erfindung weiter:

Beispiel 1

Bestimmung von Albumin

1. Herstellung der Verdrängungsmatrix

a) Vernetzung von Humanserum-Albumin (HSA) mittels Disuccinidylsuberat (DSS) zu poly-Humanserum-albumin (pHSA)

1,5 g HSA werden in 30 ml Kaliumphosphat-Puffer, 200 mM, pH = 8,0 vorgelegt und innerhalb 2 Stunden mit 2,5 ml einer Lösung aus 50 mg DSS/ml Dioxan versetzt. Nach Ablauf der Vernetzungsreaktion wird gegen das 500fache Volumen Kaliumphosphat-Puffer, 20 mM, pH 7,2 dialysiert. Die hochmolekulare Fraktion (pHSA) mit einem Molekulargewicht von mehr als 650 000 Dalton wird an Superose 6® (Pharmacia, Freiburg, Bundesrepublik Deutschland) über Gelfiltration abgetrennt und nach Zugabe von 6 mg Saccharose/mg Protein lyophilisiert.

b) Immobilisierung von Humanserum-Albumin

6 x 8 mm große und 0,5 mm dicke Vliesstücke aus 50 % Polyester/50 % Linters werden mit 15 $\mu$l einer Lösung von 30 mg/l pHSA in 10 mM Natriumphosphat-Puffer pH 7,5 getränkt und 30 Minuten bei 50 °C getrocknet.

c) Konjugate aus Antikörpern gegen HSA und $\beta$-Galactosidase

Die Konjugate I bis XI wurden aus IgG (Klon 1 (I-III) oder 2 (IV-VII) ) bzw. Fab (Klon 3 (VIII-XI) ) nach der Vorschrift von T. Kitiwaga (Enzyme Immunoassay, Hrsg. Ishikawa, Kawai, Migui; Igaku Shoin Tokyo/New York 1981, S. 81-89) hergestellt und jeweils durch Superose -6®-Chromatographie fraktioniert.

Einführung von Maleimido-Gruppen in IgG

a) Zugabe von 5o $\mu$l 0.9 g/l 3-Maleimidobenzoyl-N-hydroxysuccinimidester in N,N-Dimethylformamid zu einer Lösung von 1.4 mg (9.3 mmol)IgG in 0.5 ml Natriumphosphatpuffer (0.1 mol/l; pH 7.0).
b) Inkubation bei 30°C für 30 min. Chromatographie an Sephadex G-25® (1x45 cm) mit 0.1 mol/l Natriumphosphatpuffer pH 6.5 als Elutionsmittel.

Konjugation mit $\beta$-Galactosidase

a) Lösen von 1.5 mg (2.8 mmol) $\beta$-Galactosidase (lyophilisiert) in 1.4 ml Natriumphosphatpuffer (0.1 mol/l; pH 6.5) enthaltend 1.25 mg (8,3 mmol) Maleimido-IgG. Endkonzentration von IgG und Enzym ist 6 bzw. 2 mmol/l.
b) Inkubation 20 h bei 4°C.
c) Trennung an Sepharose 6 B® (1.5 x 45 cm Säule) mit Elutionsmittel folgender Zusammensetzung:

| | |
|---|---|
| Natriumphosphat, pH 6.5 | 10 mmol/l |
| Natriumchlorid | 0.1 mol/l |
| Magnesiumchlorid | 1 mmol/l |
| Natriumazid | 1 g/l |

d) Ablesen der Absorption des Eluats bei 280 mm.
e) Fraktionierung in pools nach Absorptionsprofil
f) Bestimmung der $\beta$-Galactosidase-Aktivität der pools.
Diese Methode ist auch in E. Ishikawa, J. Immunoassay 4 (3), 209-237 (1983) beschrieben.

```
I     ca.  4 - 7  IgG pro Molekül  ß-Galactosidase
II         2 - 5                "
III        1 - 2                "
IV         4 - 7                "
V          3 - 5                "
VI         2 - 3                "
VII        1 - 2                "
VIII       8 - 14 Fabfragmente pro Molekül ß-Galctosidase
IX         6 - 10               "
X          4 - 8                "
XI         1 - 4                "
```

d) Verdrängungsmatrix

Es wurden 15 $\mu$l einer Lösung des Konjugats (4 U/ml) aus c) in 0.1 M HEPES-Puffer (pH 7,5) mit 0.5 % Rinderalbumin auf ein Vlies aus b) aufgetropft. Anschließend wurde das Vlies getrocknet.

e) Bestimmung des Leerwerts und des Meßbereichs

Ein Stapel von zwei 6 x 8 mm größen Vliesen aus d) wird mit 55 µl Pufferlösung (50 mmol/l Phosphat pH 7,5) mit

A) 0 mg/l humanem Serumalbumin (HSA)

B) 100 mg/l humanem Serumalbumin (HSA)

getränkt. Nach 5 Minuten wird die Flüssigkeit von der Matrix abzentrifugiert. Man gibt zu der Flüssigkeit 5 mmol/l Chlorphenolrot-β-galactosid und mißt die Zunahme der Extinktion bei 576 nm in einer Küvette bei 37 °C mittels eines Photometers.

Aus den Meßwerten A) ergibt sich der Leerwert, d. h. der Wert, der auch ohne Anwesenheit des Analyten ein Signal vortäuscht.

Die Meßwerte zu B) entsprechen der Signalgröße für 100 mg/l Analyt.

Der Quotient aus Signalgröße zu Leerwert ist ein Maß für die erreichbare Genauigkeit des Tests.

Tabelle 1 gibt den Leerwert (A), den Meßwert (B) und den Quotienten B/A für die einzelnen Konjugatmischungen I bis XI wieder:

| Konjugat | Leerwert (A) [mE/min] | Meßwert (B) [mE/min] | B/A |
|----------|-----------------------|----------------------|------|
| I | 55 | 1190 | 21.6 |
| II | 175 | 1900 | 10.8 |
| III | 520 | 2370 | 4.5 |
| IV | 70 | 940 | 13.4 |
| V | 70 | 1385 | 19.7 |
| VI | 385 | 2175 | 5.6 |
| VII | 1330 | 2300 | 1.7 |
| VIII | 80 | 580 | 7.2 |
| XI | 106 | 660 | 6.2 |
| X | 180 | 1000 | 5.5 |
| XI | 310 | 1060 | 3.4 |

Es ist klar ersichtlich, daß mit den markierten Antikörpern, die die meisten Bindungsstellen aufweisen, die beste Genauigkeit erreicht werden kann.

2. Aufnahme von Eichkurven zur Bestimmung von Albumin

6 x 8 mm große und 0.5 mm dicke Vliesstücke aus 50 % Polyester/50 % Linters werden mit 15 µl einer Lösung von 50 bzw. 100 mg/l pHSA in 0.01 mmol/l Phosphatpuffer pH 7.25 getränkt und getrocknet. Danach werden die Vliese jeweils mit 15 µl einer Lösung von 4 U/ml des markierten Antikörpers I in HEPES-Puffer (100 mmol, pH 7.5)+ 0.5% RSA getränkt und getrocknet.

Beide derartig hergestellte Vliese B und C sind zur Albuminbestimmung geeignet. Zur Aufnahme ihrer Eichkurven wurden sie mit Proben, die 0 mg/l, 10 mg/l, 50 mg/l bzw. 100 mg/l HSA enthielten, getränkt. Nach fünf Minuten wurde die Flüssigkeit vom Vlies durch Zentrifugation getrennt und ihr 5 mmol/l Chlorphenolrot-β-galactosid (CPRG) zugegeben. Die Extinktionszunahme E (mE/min) wurde photometrisch wie unter e) bestimmt.

Die Eichkurven für die Vliese B und C sind in Fig. 2 abgebildet. Kurve I gibt den Extinktionsverlauf für das mit 50 mg/l pHSA getränkte Vlies B wieder und Kurve II wurde mit dem mit 100 mg/l pHSA getränkten Vlies C erhalten.

3. Bestimmung eines unbekannten Albumingehalts

7

Zur Bestimmung von Albumin wird ein Vlies B oder C mit 25 µl Probe unbekannten Analytgehalts versetzt; nach 5 Minuten wird die Probenflüssigkeit entfernt, CPRG zugegeben und ebenfalls die Extinktionszunahme E gemessen. Aus dem erhaltenen Wert läßt sich mittels der Eichkurve auf den Albumingehalt schließen.

Beispiel 2

Bestimmung von $\alpha_1$-Mikroglobulin

Herstellung der Verdrängungsmatrix

a) Herstellung von mit thermo RSA-Streptavidin überzogenem Vlies

Thermisch aggregiertes RSA, im folgenden als Thermo-RSA bezeichnet, wurde in folgender Weise hergestellt: 1 g RSA wurde in 100 ml 50 mmol Kaliumphosphatlösung bei einem pH von 7,0 gelöst, auf 70°C erhitzt und 4 Stunden unter leichtem Rühren bei dieser Temperatur gehalten. Die Lösung wurde abgekühlt, filtriert und auf eine Konzentration von 50 mg/ml eingestellt. Anschließend wurde gegen das 30fache Volumen bidest. Wasser dialysiert.

Herstellung eines Konjugats von Streptavidin mit Thermo-RSA: Aus Streptomyces avidinii gewonnenes Streptavidin wurde mit Maleimido-hexanoyl-N-hydroxy-succinimid umgesetzt, und man erhielt auf diese Weise ein Maleimidogruppen tragendes Streptavidin. Thermo-RSA wurde mit S-Acetylmercaptobernsteinsäureanhydrid umgesetzt und anschließend die geschützten SH-Gruppen durch Zugabe von Hydroxylamin freigesetzt. Das Maleimidogruppen enthaltende Streptavidin wurde sodann mit dem SH-Gruppen enthaltenden Thermo-RSA vermischt unter Bildung des gewünschten Konjugats.

6x8 mm große und 0,5 mm dicke Vliesstücke aus 50% Polyester/50% Linters werden mit 15 µl einer Lösung von 200 mg/l Thermo-RSA-Streptavidin in 10 mM Natriumphosphatpuffer pH 7,5 getränkt und 30 Minuten bei 50°C getrocknet.

b) Herstellung von biotinyliertem $\alpha_1$-Mikroglobulin analog Vorschrift Biotinylierung monoklonaler Antikörper (gemäß Peters, Baumgarten, Schulze: Monoklonale Antikörper, Herstellung und Charkaterisierung; Verl. Springer 1985).

c) Konjugate aus monoklonalem AK gerichtet gegen $\alpha_1$ M und $\beta$-Galaktosidase.

Das Konjugat wurde nach der Vorschrift von T. Kitiwaga in Enzyme Immunoassay (Eds. Ishikawa, Kuwai, Migui; Igaku Shoin Tokyo/New York (1981) pp 81-89) hergestellt und mit Superose™6-Chromatographie in die Pools I und II fraktioniert.

Pool I ca. 3-7 IgG/$\beta$ Gal

Pool II ca. 1-3 IgG/$\beta$ Gal

d) Verdrängungsmatrix

Es wurden 15 µl einer Lösung von 50 mg/l biotinylierten $\alpha_1$-Mikroglobulins in 10 mmol/l Phosphatpuffer pH 7,5 auf ein Vlies aus a) aufgetropft. Anschließend wurde das Vlies getrocknet. Danach wurden 15 µl einer Lösung von 4 U/ml Konjugat I (Vlies D) bzw. von Konjugat II (Vlies E) in 0.1 mol/l HEPES-Puffer (pH 7.5) mit 0.5 % RSA aufgetropft.

Wie in Beispiel 1 wurden der Leerwert A bzw. die Signalgröße B bei der Bestimmung mit Proben

A 0 mg $a_1$M/l

B 100 mg $a_1$M/l

bestimmt (siehe Tabelle 2)

| Konjugat | Leerwert (A) [mE/min] | Meßwert (B) [mE/min] | B/A |
|---|---|---|---|
| I | 175 | 803 | 4.6 |
| II | 327 | 960 | 2.9 |

Auch hier zeigt sich, daß der Leerwert mit abnehmender Anzahl der Bindungsstellen des markierten Antikörpers überproportional ansteigt.

Die Aufnahme der Eichkurve und die Bestimmung eines unbekannten $\alpha$1 Mikroglobulingehalts einer Probe erfolgen analog zu Beispiel 1.

**Patentansprüche**

1. Verfahren zur Bestimmung eines Analyten in einer flüssigen Probe durch Inkontaktbringen der Probenflüssigkeit mit einem immobilisierten Analyten oder immobilisierten Analytanalogon und einem markierten Antikörper, der sowohl mit dem zu bestimmenden Analyten als auch mit dem immobilisierten Analyten oder immobilisierten Analytanalogon eine immunologische Reaktion eingehen kann und der durch Bindung an den immobilisierten Analyten bzw. das immobilisierte Analytanalogon immobilisiert ist, dadurch gekennzeichnet, daß der markierte Antikörper pro Markierung mindestens 4 Bindungsstellen für den Analyten bzw. das Analytanalogon aufweist.

2. Verfahren gemäß Anspruch 1, dadurch gekennzeichnet, daß der Analyt in der Probe ein hochkonzentrierter Analyt ist.

3. Reagenz zum Nachweis eines Analyten in einer Probe enthaltend einen immobilisierten Analyten oder ein immobilisiertes Analytanalogon sowie einen markierten Antikörper, der sowohl mit dem zu bestimmenden Analyten als auch mit dem immobilisierten Analyten oder immobilisierten Analytanalogon eine immunologische Reaktion eingehen kann, dadurch gekennzeichnet, daß der markierte Antikörper pro Markierung mindestens 4 Bindungsstellen für den Analyten bzw. das Analytanalogon aufweist.

4. Reagenz gemäß Anspruch 3, dadurch gekennzeichnet, daß der immobilisierte Analyt oder das immobilisierte Analytanalogon über Streptavidin/Biotin-Wechselwirkungen an ein Vlies gebunden ist.

5. Teststreifen zum Nachweis der Anwesenheit oder der Menge eines Analyten in einer flüssigen Probe, enthaltend ein saugfähiges Vlies, das einen immobilisierten Analyten oder ein immobilisiertes Analytanalogon und einen daran gebundenen markierten Antikörper enthält, der sowohl mit dem Analyten, als auch mit dem Analytanalogon eine immunologische Reaktion eingehen kann, und ein Vlies, in dem die Menge an markiertem Antikörper gemessen werden kann, dadurch gekennzeichnet, daß der markierte Antikörper pro Markierung mindestens 4 Bindungsstellen für den Analyten bzw. das Analytanalogon aufweist.

**Claims**

1. Process for the determination of an analyte in a liquid sample by bringing into contact of the sample liquid with an immobilised analyte or immobilised analyte analogue and a labelled antibody which can enter into an immunological reaction not only with the analyte to be determined but also with the immobilised analyte or immobilised analyte analogue and which is immobilised by binding to the immobilised analyte or the immobilised analyte analogue, characterised in that the labelled antibody has, per labelling, at least 4 binding positions for the analyte or the analyte analogue.

2. Process according to claim 1, characterised in that the analyte in the sample is a highly concentrated analyte.

3. Reagent for the detection of an analyte in a sample containing an immobilised analyte or an immobilised analyte analogue, as well as a labelled antibody which can enter into an immunological reaction not only with the analyte to be determined but also with the immobilised analyte or immobilised analyte analogue, characterised in that the labelled antibody has, per labelling, at least 4 binding positions for the analyte or the analyte analogue.

4. Reagent according to claim 3, characterised in that the immobilised analyte or the immobilised analyte analogue is bound to a fleece via streptavidin/ biotin interactions.

5. Test strip for the detection of the presence or of the amount of an analyte in a liquid sample, containing an absorbent fleece which contains an immobilised analyte or an immobilised analyte analogue and a labelled antibody bound thereon which can enter into an immunological reaction not only with the analyte but also with the analyte analogue in which the amount of labelled antibody can be measured, characterised in that the labelled antibody has, per labelling, at least 4 binding positions for the analyte or the analyte analogue.

## Revendications

1. Procédé de détermination d'un analyte dans un échantillon liquide, par mise en contact de l'échantillon liquide avec un analyte fixé ou un analogue d'analyte fixé et un anticorps marqué qui peut entrer en réaction immunologique avec l'analyte à déterminer aussi bien qu'avec l'analyte fixé ou l'analogue d'analyte fixé, et qui est fixé par liaison avec l'analyte fixé ou l'analogue d'analyte fixé, caractérisé en ce que l'anticorps marqué présente, par marqueur, au moins 4 sites de liaison pour l'analyte ou l'analogue d'analyte.

2. Procédé selon la revendication 1, caractérisé en ce que l'analyte est présent dans l'échantillon à une concentration élevée.

3. Réactif pour la détermination d'un analyte dans un échantillon contenant un analyte fixé ou un analogue d'analyte fixé ainsi qu'un anticorps marqué, qui peut entrer en réaction immunologique avec l'analyte à déterminer aussi bien qu'avec l'analyte fixé ou l'analogue d'analyte fixé, caractérisé en ce que l'anticorps marqué présente, par marqueur, au moins 4 sites de liaison pour l'analyte ou l'analogue d'analyte.

4. Réactif selon la revendication 3, caractérisé en ce que l'analyte fixé ou l'analogue d'analyte fixé est immobilisé sur un non-tissé par interaction streptavidine/biotine.

5. Bande de test pour la détection qualitative ou quantitative d'un analyte dans un échantillon liquide, contenant un non-tissé absorbant, qui comprend un analyte fixé ou un analogue d'analyte fixé et un anticorps marqué fixé sur celui-ci, qui peut entrer en réaction immunologique avec l'analyte aussi bien qu'avec l'analogue d'analyte, et un non-tissé, dans laquelle la quantité d'anticorps marqué peut être déterminée, caractérisée en ce que l'anticorps marqué présente, par marqueur, au moins 4 sites de liaison pour l'analyte ou l'analogue d'analyte.

FIG. 1

FIG. 2

FIG. 3